(19) Europäisches Patentamt
European Patent Office
Office européen des brevets

(11) Veröffentlichungsnummer: **0 523 480 B1**

(12) **EUROPÄISCHE PATENTSCHRIFT**

(45) Veröffentlichungstag der Patentschrift: **04.10.95**

(51) Int. Cl.[6]: **C08F 8/30**, C08F 8/44

(21) Anmeldenummer: **92111341.1**

(22) Anmeldetag: **03.07.92**

(54) **Polyacrylsäureester mit Ammonium- und langkettigen Kohlenwasserstoffoxy-Gruppen.**

(30) Priorität: **16.07.91 DE 4123478**

(43) Veröffentlichungstag der Anmeldung:
**20.01.93 Patentblatt 93/03**

(45) Bekanntmachung des Hinweises auf die Patenterteilung:
**04.10.95 Patentblatt 95/40**

(84) Benannte Vertragsstaaten:
**BE CH DE ES FR GB IT LI NL**

(56) Entgegenhaltungen:
**EP-A- 0 293 750**
**EP-A- 0 373 494**
**EP-A- 0 386 507**
**FR-A- 2 528 435**
**GB-A- 1 023 281**

(73) Patentinhaber: **Th. Goldschmidt AG**
**Goldschmidtstrasse 100**
**D-45127 Essen (DE)**

(72) Erfinder: **Fock, Jürgen, Dr.**
**Mörsenbroicher Weg 114**
**W-4000 Düsseldorf 30 (DE)**
Erfinder: **Leidreiter, Holger, Dr.**
**Reuenberg 98 a**
**W-4300 Essen 11 (DE)**
Erfinder: **Esselborn, Eberhard**
**Pilotystrasse 21**
**W-4300 Essen 1 (DE)**

**EP 0 523 480 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Polyacrylsäureester mit Ammonium- und langkettigen Kohlenwasserstoffoxy-Gruppen und deren Verwendung als Mittel, welche gleichzeitig auf wäßrige Lösungen verdickend wirken und antistatische Eigenschaften aufweisen und deshalb besonders nützlich zur Herstellung kosmetischer Produkte, insbesondere zur Haarpflege, sind.

Die Erfindung betrifft insbesondere Polyacrylsäureester mit einem verringerten Gehalt an niedrigmolekularen oder oligomeren Verbindungen sowie einer Verteilung der Polymeren, welche der Poisson-Verteilung angenähert ist, und insbesondere solche Polyacrylsäureester, die verbesserte physiologische Eigenschaften aufweisen.

Polyacrylsäureester mit quaternären Ammoniumgruppen sind bekannt und werden als kationische Hilfsmittel zur antistatischen Ausrüstung von Textilfasern (DE-A-22 42 914), zur Herstellung elektrisch leitender Kopierpapiere oder als Haarfestiger (DE-A-24 23 182) verwendet. Diese Verbindungen werden durch Copolymerisation von Acrylsäureestern, insbesondere Acrylsäuremethylestern und Acrylsäureesterderivaten, welche quaternäre Ammoniumgruppen aufweisen, hergestellt. Die radikalische Copolymerisation führt jedoch aufgrund der unterschiedlichen Copolymerisationsparameter und insbesondere durch den ionischen Charakter der Monomeren mit quaternären Ammoniumgruppen zu Polymeren mit einer weiten Molekulargewichtsverteilung der Copolymerisate.

Dieser Nachteil wird durch ein Umesterungsverfahren vorgebildeter Polyacrylsäurealkylester überwunden. So sind aus der DE-PS 38 42 201 Polyacrylsäureester mit quaternären Ammoniumgruppen bekannt, die erhältlich sind durch Umesterung von durch radikalische Polymerisation erhaltenen Polyacrylsäurealkylestern, wobei der Alkylrest 1 bis 4 Kohlenstoffatome enthält, mit einem Gemisch von

a) Dialkylaminoalkanolen der allgemeinen Formel

$HO\text{-}R^1\text{-}NR^2R^3$

wobei

$R^1$ ein zweiwertiger Alkylenrest mit 2 bis 4 Kohlenstoffatomen oder ein zweiwertiger Dialkylenetherrest mit 4 bis 6 Kohlenstoffatomen ist und

$R^2$, $R^3$ gleich oder verschieden sind und Alkylreste mit 1 bis 4 Kohlenstoffatomen bedeuten, und

b) gesättigten oder ungesättigten aliphatischen Alkoholen mit 8 bis 22 Kohlenstoffatomen,

wobei das molare Verhältnis der Dialkylaminoalkanole und der Alkanole mit 8 bis 22 Kohlenstoffatomen 1 : 9 bis 9 : 1 beträgt,

in solchen Mengen, daß bis zu 70 % der Estergruppen umgeestert werden, in Gegenwart eines an sich bekannten Umesterungskatalysators, bei Temperaturen von 70 bis 140 °C, gegebenenfalls in Gegenwart eines Lösungsmittels und nachfolgende Quaternierung mit Alkyl- oder Alkylarylhalogeniden der allgemeinen Formel

$R^4X$

wobei

$R^4$ ein Alkylrest mit 1 bis 4 Kohlenstoffatomen oder ein Benzylrest und

X ein Halogenatom ist,

oder mit Dimethyl- oder Diethylsulfat in an sich bekannter Weise bei Temperaturen von 20 bis 140 °C und gegebenenfalls erhöhtem Druck.

Die gemäß der DE-A-38 42 201 hergestellten Produkte enthalten nur geringe Anteile niedermolekularer Verbindungen und sind deshalb physiologisch wesentlich unbedenklicher.

Aus der älteren Patentanmeldung DE-A-40 06 093.4 sind Polyacrylsäureester mit langkettigen Kohlenwasserstoff- und Polyoxyalkylengruppen bekannt, welche einerseits als Emulgatoren, Solubilisierungsmittel und zum Verdicken wäßriger, aniontensidhaltiger Zubereitungen verwendet werden können und deren physiologische Eigenschaften andererseits in ähnlicher Weise, wie bereits oben beschrieben, verbessert sind.

Diese Verbindungen sind durch Umesterung von durch radikalische Polymerisation erhaltenen Polyacrylsäurealkylestern, deren Alkylgruppen 1 bis 8 Kohlenstoffatome aufweisen, erhältlich, wobei bis zu 50 % der Acrylsäureester durch die entsprechenden Methacrylsäureester ersetzt sein können, mit Polyoxyalkylenmonoolen der allgemeinen durchschnittlichen Formel

$R^1O\text{-}(C_nH_{2n}O\text{-})_xH$

wobei

- $R^1$ ein Alkylrest mit 8 bis 30 Kohlenstoffatomen, ein Alkenylrest mit 8 bis 22 Kohlenstoffatomen oder ein Mono- oder Dialkylphenylrest mit 6 bis 16 Kohlenstoffatomen je Alkylrest ist, wobei bis zu 40 % der Reste $R^1$ durch Alkylreste mit 1 bis 4 Kohlenstoffatomen ersetzt sein können,
- n einen Wert von 2, 3 oder 4 hat und im durchschnittlichen Molekül einen mittleren Wert von 2,0 bis 2,5 aufweist, und
- x einen Wert von 10 bis 200 hat,

in solchen Mengen, daß 5 bis 70 % der Estergruppen umgeestert werden und in Gegenwart eines an sich bekannten Umesterungskatalysators, bei Temperaturen von 70 bis 170 °C und gegebenenfalls in Gegenwart eines Lösungsmittels.

Die vorliegende Erfindung befaßt sich mit dem technischen Problem, Polyacrylsäureester herzustellen, welche die hervorragenden anwendungstechnischen Eigenschaften der Polymerisate mit quaternären Ammoniumgruppen gemäß der DE-A-38 42 201 aufweisen, ohne aber wie diese Verbindungen auf wäßrige Zubereitungen verdünnend zu wirken, sondern vielmehr verdickende und emulgierende Eigenschaften besitzen, wie sie den Verbindungen der älteren Patentanmeldung DE-A-40 06 093 eigen sind. Eine Mischung von wäßrigen Lösungen der beiden Copolymerisate brachte nicht den gewünschten Erfolg, da häufig struktur- und/oder konzentrationsabhängige Entmischungen auftraten und die Mischungen anwendungstechnisch unbrauchbar waren. Überraschenderweise zeigte sich aber, daß es möglich ist, die gewünschten Eigenschaften innerhalb eines Copolymerisates durch gemeinsame Umesterung eines Polyacrylsäurealkylesters mit einem Gemisch von Aminoalkoholen bzw. -Hydroxyethylmorpholin und Polyoxyalkylenmonoolen und anschließende Quaternierung der tertiären Aminogruppen zu erhalten.

Gegenstand der vorliegenden Erfindung sind deshalb Polyacrylsäureester mit Ammonium- und langkettigen Kohlenwasserstoffoxy-Gruppen, erhältlich durch Umesterung von durch radikalische Polymerisation erhaltenen Polyacrylsäurealkylestern, deren Alkylreste 1 bis 8 Kohlenstoffatome aufweisen, wobei bis zu 50 % der Acrylsäureester durch die entsprechenden Methacrylsäureester ersetzt sein können, mit

a) tertiären Aminen der allgemeinen Formel

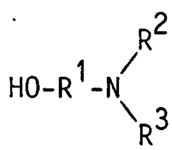 I oder 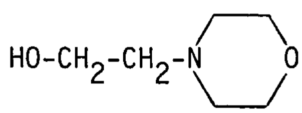 II

$R^1$ = zweiwertiger Rest der allgemeinen Formel $-(CH_2)_q-$,

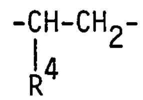

oder $-(C_nH_{2n}O)_m-C_pH_{2p}-$

- $R^4$ = Alkylrest mit 1 bis 16 Kohlenstoffatomen,
- q = 2, 3 oder 4,
- n = 2, 3 oder 4,
- m = 1 bis 20,
- p = 2, 3 oder 4,
- $R^2$, $R^3$ = jeweils Alkylrest mit 1 bis 18 Kohlenstoffatomen und

b) Polyoxyalkylenmonoolen der allgemeinen durchschnittlichen Formel

$R^5O-(C_rH_{2r}O-)_sH$ III

$R^5$ = Alkylrest mit 8 bis 30 Kohlenstoffatomen, der im Polymeren gleich oder verschieden sein kann, oder Alkenylrest mit 8 bis 22 Kohlenstoffatomen, oder Mono- oder Dialkylphenylrest mit 6 bis 16 Kohlenstoffatomen je Alkylrest,

wobei bis zu 40 % der Reste $R^5$ durch Alkylreste mit 1 bis 4 Kohlenstoffatomen ersetzt sein können,

r = 2, 3 oder 4 und im durchschnittlichen Molekül 2,0 bis 2,5,

s = 10 bis 100,

wobei das molare Verhältnis der tertiären Amine zu den Polyoxyalkylenmonoolen 1 : 9 bis 9 : 1 beträgt, in solchen Mengen, daß 5 bis 70 % der Estergruppen umgeestert werden, in Gegenwart eines an sich bekannten Umesterungskatalysators, bei Temperaturen von 70 bis 170 °C, gegebenenfalls in Gegenwart eines Lösungsmittels, und anschließende (1) Quaternierung der Umesterungsprodukte mit Alkyl- oder Arylhalogeniden der allgemeinen Formel $R^6X$, wobei $R^6$ ein Alkylrest mit 1 bis 4 Kohlenstoffatomen oder ein Benzylrest und X ein Halogenrest ist, oder mit Dimethyl- oder Diethylsulfat in an sich bekannter Weise bei Temperaturen von 20 bis 140 °C und gegebenenfalls erhöhtem Druck oder (2) Protonierung der Umesterungsprodukte durch Umsetzung mit anorganischen oder organischen Säuren.

Die Reste $R^1$ bis $R^5$ und die Indices werden im folgenden noch näher erläutert:

$R^1$ ist ein zweiwertiger Rest der Formel $-(CH_2)_q-$,

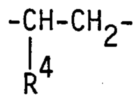

oder $-(C_nH_{2n}O)_m-C_pH_{2p}-$.

Beispiele solcher Reste sind $-(CH_2)_4-$;

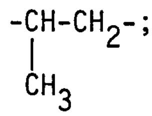

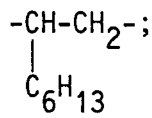

$-(CH_2)_2O(CH_2)_2-$; $-(CH_2)_3O(CH_2)_2-$;
$-(CH_2)_2O(CH_2)_2O(CH_2)_3-$;

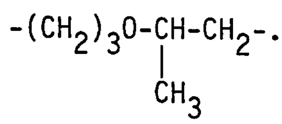

Besonders bevorzugt sind $-(CH_2)_q$-Reste sowie die Reste

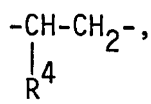

bei denen $R^4$ ein Methyl- oder Ethylrest ist. Bei den Etherresten sind solche bevorzugt, bei denen n und p einen Wert von 2 oder 3 haben. m hat vorzugsweise einen Wert von 1 bis 10.

$R^2$ und $R^3$ sind jeweils Alkylreste mit 1 bis 18 Kohlenstoffatomen. Die Alkylreste können geradkettig oder verzweigt sein. Bevorzugt sind Alkylreste mit 1 bis 6 Kohlenstoffatomen. Insbesondere bevorzugt sind Alkylreste mit 1 bis 4 Kohlenstoffatomen, wie der Methyl-, Ethyl-, Propyl-, iso-Propyl, Butyl- und iso-Butylrest.

Innerhalb des polymeren Moleküls können die Reste und Indices verschiedene Bedeutung bzw. Werte annehmen. Hierdurch bedingt können im durchschnittlichen Polymerenmolekül die Indices auch gebrochene Zahlenwerte annehmen.

$R^5$ kann gleich oder verschieden sein und einen Alkylrest, einen Alkenylrest oder einen Mono- oder Dialkylphenylrest bedeuten.

Der Alkylrest hat 8 bis 30 Kohlenstoffatome. Beispiele solcher Alkylreste sind der Octyl-, Decyl-, Undecyl-, Dodecyl-, Stearyl- oder Behenylrest. Bevorzugt sind Alkylreste mit 14 bis 22 Kohlenstoffatomen

und insbesondere solche, die sich von den Fettalkoholen herleiten, die aus natürlichen Fettsäuren durch Reduktion erhalten worden sind.

Der Alkenylrest hat 8 bis 22 Kohlenstoffatome, vorzugsweise 8 bis 18 Kohlenstoffatome. Besonders bevorzugt ist der Alkenylrest eines ungesättigten Fettalkohols, wie etwa der Oleyl- oder Linoleylrest.

Der Mono- oder Dialkylphenylrest weist in der oder den Alkylgruppen jeweils 6 bis 16, vorzugsweise 6 bis 12, Kohlenstoffatome auf. Bevorzugte Alkylphenylreste sind der Mono- oder Dioctyl-, -nonyl-, -decyl- oder -dodecylphenylrest.

Unabhängig von diesen Definitionen von $R^5$ können jedoch bis zu 40 % der Reste $R^5$ durch Alkylreste mit 1 bis 4 Kohlenstoffatomen ersetzt sein.

In der allgemeinen Formel III gibt r die Anzahl der Kohlenstoffatome der Oxyalkylengruppen an. r hat dabei je Oxyalkyleneinheit einen Wert von 2, 3 oder 4. Im durchschnittlichen Molekül hat r einen mittleren Wert von 2,0 bis 2,5, vorzugsweise von 2,0 bis 2,3.

Der Index s entspricht der Anzahl der Oxyalkyleneinheiten und hat einen Wert von 10 bis 200, vorzugsweise von 40 bis 60. Auch hierbei handelt es sich um durchschnittliche Werte.

Das Verhältnis der aus Aminoalkohol I bzw. $\beta$-Hydroxyethylmorpholin II bestehenden Umesterungskomponente a) zur Komponente b), die vom Polyoxyalkylenmonool gebildet wird, beträgt 1 : 9 bis 9 : 1, vorzugsweise 1 : 3 bis 3 : 1.

Die Umesterung wird mit solchen Mengen der Umesterungskomponenten a) und b) durchgeführt, daß 5 bis 70 %, vorzugsweise 20 bis 50 % der Estergruppen umgeestert werden. Dies kann durch Bestimmung der Menge des bei der Umesterung freigesetzten Alkohols des Polyacrylsäureesters kontrolliert werden.

Die Umesterung erfolgt in Gegenwart eines Umesterungskatalysators. Derartige Katalysatoren sind dem Fachmann bekannt. Beispiele solcher Katalysatoren sind i-Propyl- oder n-Butyltitanat, Kalium- oder Natriummethylat, p-Toluolsulfonsäure, Methansulfonsäure oder Trifluoressigsäure.

Es ist meist vorteilhaft, die Umesterung in einem Lösungsmittel durchzuführen. Bevorzugte Lösungsmittel sind Toluol oder Xylol.

Die Umesterungstemperatur beträgt etwa 70 bis 170 °C, insbesondere 70 bis 150 °C.

Die Umesterungsprodukte werden in an sich bekannter Weise quaterniert oder protoniert.

Die Quaternierung erfolgt mit Alkyl- oder Arylhalogeniden $R^6X$, wobei $R^6$ ein Alkylrest mit 1 bis 4 Kohlenstoffatomen oder ein Benzylrest und X ein Halogenrest ist. Bevorzugt sind dabei Methyl- und Benzylchlorid. Die Quaternierung wird innerhalb eines Temperaturbereiches von 20 bis 140 °C und gegebenenfalls erhöhtem Druck durchgeführt.

Anstelle einer Quaternierung kann auch eine Protonierung der tertiären Aminogruppen erfolgen. Die Protonierung wird durch Umsetzung mit anorganischen oder organischen Säuren vorgenommen. Beispiele geeigneter Säuren sind die Phosphorsäure, Essigsäure, Propionsäure und Ölsäure.

Zur Herstellung der erfindungsgemäßen Polyacrylsäureester mit Ammonium- und langkettigen Kohlenwasserstoffoxy-Gruppen durch Umesterung werden Polyacrylsäurealkylester eines mittleren Molekulargewichtes von 800 bis 30 000 bevorzugt. Dabei können bis zu 50 % dieser Acrylsäureester durch die entsprechenden Methacrylsäureester ersetzt sein. Es ist dem Fachmann klar, daß man anstelle der Homopolymerisate auch Copolymerisate von Acrylsäureestern mit anderen polymerisierbaren Monomeren einsetzen kann, soweit sie nach Art und Menge den vorgesehenen Verwendungszweck nicht stören. Beispiele solcher Comonomeren sind Styrol, Acrylamid oder Acrylnitril.

Ein weiterer Gegenstand der Erfindung besteht in der Verwendung der erfindungsgemäßen Verbindungen als konditionierende Zusätze für kosmetische, insbesondere haarpflegende Zubereitungen, welche gleichzeitig verdickende Wirkungen haben. Sie verbessern bereits in geringen Anwendungskonzentrationen von 0,2 bis 2 Gew.-%, bezogen auf Zubereitung, die Kämmbarkeit der Haare, insbesondere nasser Haare, und verringern deren statische Aufladung. Gleichzeitig wird dabei die Viskosität der Zubereitungen erhöht. Dies ist anwendungstechnisch besonders erwünscht. Dabei werden hinsichtlich der verdickenden Wirkung solche erfindungsgemäßen Verbindungen bevorzugt, bei denen $R^5$ ein Alkyl- oder Alkenylrest mit 16 bis 22 Kohlenstoffatomen ist. Als vorteilhaft hat sich hier die Verwendung von Polyethylenoxidsegmenten mit 30 bis 60 Oxyethyleneinheiten erwiesen. Durch Einbringen relativ geringer Mengen Oxypropyleneinheiten läßt sich eine Kristallisation der erfindungsgemäßen Verbindungen vermeiden und damit eine verbesserte Handhabbarkeit erreichen.

In den folgenden Beispielen werden die Herstellung der erfindungsgemäßen Verbindungen und deren antistatische und verdickende Wirkung gezeigt.

Beispiel 1 A

Herstellung von Polymethylacrylat durch radikalische Polymerisation (nicht erfindungsgemäß)

Eine Lösung von 0,6 g Azodiisobuttersäurenitril und 20,2 g Dodecylmercaptan in 50 g Toluol und 280 g (ca. 3,25 Mol) Methylacrylat wird innerhalb von 2 h in einen mit 53 g Toluol gefüllten Reaktor gegeben; das vorgelegte Lösungsmittel hat dabei eine Temperatur von 100 °C und befindet sich unter einer Stickstoffatmosphäre. Danach werden nochmal 0,9 g Azodiisobuttersäurenitril, gelöst in 20 g Methylethylketon, innerhalb von 0,5 h nachgegeben. Schließlich wird das Reaktionsgemisch noch für 1 h bei der gleichbleibenden Temperatur von 100 °C weiter erwärmt. Nach Beendigung der Reaktion wird das Lösungsmittel abdestilliert. Es verbleibt eine farblose, viskose Flüssigkeit mit einem Brechungsindex von 1,4802. Aus der gelchromatographischen Untersuchung ergibt sich für das erhaltene Polymerisat ein numerisches Molekulargewicht $\overline{M}_n$ von 1950 und für das Gewichtsmittel des Molekulargewichtes $\overline{M}_w$ 3330; der Uneinheitlichkeitskoeffizient beträgt demnach 1,71. Der Restmonomerengehalt beträgt < 0,1 %.

Beispiele 2 A bis 4 A

Herstellung von Polymethylacrylaten unterschiedlichen Molekulargewichtes durch radikalische Polymerisation (nicht erfindungsgemäß)

Es wird verfahren wie in Beispiel 1 mit der Ausnahme, daß der Gehalt an Dodecylmercaptan gesenkt wird. In Tabelle 1 wird die Abhängigkeit der Zahlen- und Gewichtsmittel des Molekulargewichtes vom Gehalt an Dodecylmercaptan gezeigt. Der gefundene Restmonomerengehalt beträgt in beiden Fällen < 0,1 %.

Tabelle 1

| Polymethylacrylat aus Beispiel | Dodecylmercaptan [Gew.-%] | Molekulargewicht $\overline{M}_n$ | Molekulargewicht $\overline{M}_w$ | Uneinheitlichkeitskoeffizient |
|---|---|---|---|---|
| 2 A | 13,5 | 836 | 1 302 | 1,56 |
| 3 A | 2,95 | 4 453 | 11 346 | 2,55 |
| 4 A | 0,43 | 16 750 | 68 500 | 4,09 |

Beispiel 5 A

Herstellung von Poly-n-butylacrylat durch radikalische Polymerisation (nicht erfindungsgemäß)

Es wird verfahren wie in Beispiel 1 mit dem Unterschied, daß anstelle von Methylacrylat n-Butylacrylat eingesetzt wird.

Aus der gaschromatographischen Untersuchung ergibt sich für das erhaltene Polymerisat ein numerisches Molekulargewicht $\overline{M}_n$ von 1900 und für das Gewichtsmittel des Molekulargewichtes $\overline{M}_w$ 3300; der Uneinheitlichkeitskoeffizientbeträgt demnach 1,73. Der Restmonomerengehalt wird zu < 0,1 % ermittelt.

Beispiel 6 A

Herstellung von Poly-2-ethylhexylacrylat durch radikalische Polymerisation (nicht erfindungsgemäß)

Es wird verfahren wie in Beispiel 1 mit dem Unterschied, daß anstelle von Methylacrylat 2-Ethylhexylacrylat eingesetzt wird.

Aus der gaschromatographischen Untersuchung ergibt sich für das erhaltene Polymerisat ein numerisches Molekulargewicht $\overline{M}_n$ von 1800 und für das Gewichtsmittel des Molekulargewichtes $\overline{M}_w$ 3030; der Uneinheitlichkeitskoeffizientbeträgt demnach 1,68. Der Restmonomerengehalt wird zu ca. 0,1 % ermittelt.

Beispiel 7 A

Herstellung eines Methylacrylat-Methylmethacrylat-Copolymerisates durch radikalische Polymerisation (nicht erfindungsgemäß)

Es wird verfahren wie in Beispiel 1 mit der Ausnahme, daß anstelle einer Menge von 280 g (ca. 3,25 Mol) Methylacrylat 140 g (ca. 1,61 Mol) Methylacrylat und 140 g (ca. 1,4 Mol) Methylmethacrylat eingesetzt werden. Aus der gelchromatographischen Untersuchung ergibt sich für das erhaltene Polymerisat ein numerisches Molekulargewicht $\overline{M}_n$ von 2280 und für das Gewichtsmittel des Molekulargewichtes $\overline{M}_w$ 4390; der Uneinheitlichkeitskoeffizient beträgt demnach 1,93. Der Restmonomerengehalt wird zu etwa 0,15 % ermittelt.

Beispiel 1 B

Herstellung eines Stearyloxypolyethylenoxidmonools (nicht erfindungsgemäß)

269 g (ca. 1 Mol) Stearylalkohol und 7,0 g (ca. 0,1 Mol) Kaliummethylat werden in einen Reaktor gegeben. Nach sorgfältiger Spülung mit Reinstickstoff wird auf 110 °C geheizt und 2420 g (ca. 55 Mol) Ethylenoxid so schnell zugegeben, daß die Reaktorinnentemperatur 120 °C und der Druck 6 bar nicht überschreiten. Nach vollständiger Einleitung des Ethylenoxids wird die Temperatur so lange auf 115 °C gehalten, bis daß gleichbleibender Druck das Ende der Nachreaktion anzeigt. Schließlich werden bei 80 bis 90 °C die nicht umgesetzten Monomeren unter Vakuum entfernt.

Das erhaltene Produkt wird mit Hilfe von verdünnter Phosphorsäure neutralisiert und das Wasser durch Destillation, das entstandene Kaliumphosphat durch Filtration zusammen mit einem Filterhilfsmittel entfernt. Das aus der Bestimmung der Hydroxylzahl bei einer angenommenen Funktionalität von 1 ermittelte Molekulargewicht beträgt 2500.

Beispiele 2 B bis 20 B

Herstellung verschiedener Alkyloxy- bzw. Alkylaryloxypolyalkylenoxidmonoole (nicht erfindungsgemäß)

Die Herstellung und Aufarbeitung verschiedener Alkoxylate von Alkanolen und von Alkylphenolen erfolgt grundsätzlich in der in Beispiel 1 B beschriebenen Weise durch Anlagerung der Alkylenoxide in Gegenwart alkalischer Katalysatoren und anschließende Neutralisation mit verdünnter Phosphorsäure.

Beispiele der verwendeten Startalkohole, der Alkylenoxide nach Art und Molzahl sowie die durch Bestimmung der Hydroxylzahl ermittelten Molekulargewichte befinden sich in der Tabelle 2.

Tabelle 2

| Beispiel Nr. | Starter | EO [Mol] | PO [Mol] | BO [Mol] | MG [OHZ] |
|---|---|---|---|---|---|
| 1 B | STA | 55 | - | - | 2500 |
| 2 B | OLA | 50 | 1,5* | - | 2310 |
| 3 B | OLA | 10 | - | - | 646 |
| 4 B | OLA | 25 | 19,0 | - | 2040 |
| 5 B | OLA | 40 | 8,5 | - | 2280 |
| 6 B | OLA | 44 | - | 3,7 | 2149 |
| 7 B | TFA | 2 | - | - | 352 |
| 8 B | TFA | 20 | - | - | 1152 |
| 9 B | TFA | 50 | 1,5* | - | 2040 |
| 10 B | BHA | 50 | 1,5* | - | 2290 |
| 11 B | C21 | 50 | 1,5* | - | 2377 |
| 12 B | CTA | 50 | 1,5* | - | 2440 |
| 13 B | DDO | 50 | 1,5* | - | 2226 |
| 14 B | OCA | 20 | 1,5* | - | 967 |
| 15 B | MEA | 21 | 1,5* | - | 949 |
| 16 B | MEA | 43 | 1,5* | - | 1970 |
| 17 B | NP | 10 | - | - | 660 |
| 18 B | NP | 50 | - | - | 2550 |
| 19 B | DNP | 49 | - | - | 1982 |
| 20 B | C30 | 100 | - | - | 4800 |

* Propylenoxid am hydroxyfunktionellen Kettenende

Legende:

MEA = Methanol
OCA = Octanol-1
DDO = Dodecanol-1
CTA = Cetylalkohol
OLA = Oleylalkohol
TFA = Talgfettalkohol
STA = Stearylalkohol
C21 = Exxal-21 der Fa. Esso
BHA = Behenylalkohol
NP = Nonylphenol
DNP = Dinonylphenol
C30 = $C_{30}$-Alkohol

Beispiel 1 C

Herstellung eines Umesterungsproduktes aus Polymethylacrylat, alkoxyliertem Stearylalkohol und Diethylaminoethanol

92,3 g des Polymethylacrylates aus Beispiel 1 A, gelöst in 23,1 g Ethanol/MEK 7 : 3, werden zusammen mit 250 g (0,1 Mol) eines Stearyloxypolyethers gemäß Beispiel 1 B und 46,8 g (ca. 0,4 Mol) Diethylaminoethanol unter Stickstoff erhitzt. Nach Zugabe von 380 g aromatenfreiem Benzin (Isopar E der Fa. Exxon; Siedebereich 115 bis 130 °C) wird das Ethanol/MEK-Gemisch und Spuren von Wasser durch Fraktionierung abgetrennt. Anschließend erfolgt die Zugabe von 1,0 g Isopropyltitanat. Das bei der Umesterung entstehende Methanol wird durch Fraktionierung abgeschieden. Innerhalb von 3 bis 4 h werden nochmals 3,0 g Isopropyltitanat in drei gleichen Portionen zugegeben. Die Reaktion ist nach etwa 6 h beendet; das Ende wird durch eine Kopftemperatur von etwa 118 bis 120 °C angezeigt.

Der durch gelchromatographische Untersuchung ermittelte Anteil an nicht umgesetztem Stearyloxypolyether beträgt 22 g, was einem Umsatz von 91 % entspricht. Der gaschromatographisch ermittelte Anteil an nicht umgesetztem Diethylaminoethanol beträgt 2,8 g, was einem Umsatz von 94 % entspricht. Der Methanolgehalt im Destillat wurde zu 15,0 g ermittelt, was einem Unsatz von 94 % entspricht. Die Farbzahl des Produktes nach Gardner beträgt nach Entfernung des Umesterungskatalysators durch Hydrolyse und Filtration 1 bis 2.

Beispiele 2 C bis 32 C

Umesterungen von verschiedenen Polyacrylsäureestern mit verschiedenen Fettalkoholalkoxylaten und Alkanolen mit tertiären Aminogruppen

Es wird verfahren wie in Beispiel 1 C mit der Ausnahme, daß Polyacrylsäureester unterschiedlichen Molekulargewichtes und unterschiedlicher Kohlenstoffzahl sowie in einem Fall ein Methylacrylat-Methylmethacrylat-Copolymerisat als estergruppenhaltige Komponente (s. Beispiele 1 A bis 7 A) zum Einsatz kommen. Weiterhin werden unterschiedliche Fettalkoholalkoxylate verwendet, die sich in bezug auf die Kohlenstoffzahl des Alkyl-, Alkylacyl- bzw. Alkenylrestes, auf das Molekulargewicht des Polyethersegmentes und dessen Zusammensetzung unterscheiden (s. Beispiele 1 B bis 20 B). Gleichzeitig werden bei der Umesterung verschiedene Dialkylaminoalkanole, Dialkylaminooligoalkylenglykole bzw. in einem Fall Hydroxyethylmorpholin eingesetzt.

Der Substitutionsgrad der Theorie bezeichnet den Quotienten aus der Zahl der zu substituierenden Estergruppen und der Zahl der ursprünglich vorhandenen Estergruppen. Der Substitutionsgrad der Praxis, d.h. der Quotient aus der Zahl der tatsächlich substituierten Estergruppen zur Zahl der zu substituierenden Estergruppen, ergibt sich aus der Menge an abgeschiedenem niedermolekularen Alkohol bzw. aus der gelchromatographischen Untersuchung. Art und Menge der Komponenten sowie theoretischer und praktischer Substitutionsgrad sind in Tabelle 3 angegeben. Bei Verwendung von Poly-2-ethylhexylacrylat wird die Umesterung ohne Einsatz eines Lösungsmittels bei vermindertem Druck durchgeführt.

Beispiele 33 C und 34 C

Herstellung von Umesterungsprodukten unter Verwendung zweier Polyalkylenoxidmonoole und Diethylaminoethanol

Es wird verfahren wie in Beispiel 1 C mit der Ausnahme, daß zwei unterschiedliche Polyethermonoole gleichzeitig zum Einsatz kommen. Art und Menge des Polyalkylenoxidmonools sowie der theoretische und praktische Substitutionsgrad sind in Tabelle 3 angegeben.

Tabelle 3

| Beispiel | Polyacrylat | | Fettalkoholalkoxylate | | | Dialkylaminoalkanol | | | Substitutionsgrad | |
|---|---|---|---|---|---|---|---|---|---|---|
| Nr. | Beispiel Nr. | Menge [g] | Beispiel Nr. | Menge [Mol] | Menge [g] | Art | Menge [Mol] | Menge [g] | theor. [%] | prakt. [%] |
| 1 C | 1 A | 92,3 | 1 B | 0,10 | 250,0 | DEAE | 0,40 | 46,90 | 50 | 94 |
| 2 C | 1 A | 92,3 | 1 B | 0,16 | 400,0 | DEAE | 0,25 | 29,30 | 50 | 97 |
| 3 C | 1 A | 92,3 | 1 B | 0,25 | 625,0 | DEAE | 0,05 | 5,85 | 30 | 96 |
| 4 C | 1 A | 92,3 | 1 B | 0,20 | 500,0 | DEAE | 0,15 | 17,55 | 35 | 98 |
| 5 C | 1 A | 92,3 | 1 B | 0,10 | 250,0 | DEAE | 0,33 | 38,60 | 43 | 99 |
| 6 C | 1 A | 92,3 | 1 B | 0,05 | 125,0 | DEAE | 0,45 | 52,65 | 50 | 95 |
| 7 C | 1 A | 92,3 | 1 B | 0,15 | 375,0 | DMAE* | 0,10 | 8,90 | 25 | 98 |
| 8 C | 3 A | 88,7 | 2 B | 0,10 | 231,0 | DMAE* | 0,50 | 44,55 | 60 | 97 |
| 9 C | 2 A | 99,5 | 3 B | 0,20 | 129,2 | DEAE | 0,30 | 35,16 | 50 | 97 |
| 10 C | 1 A | 92,3 | 4 B | 0,15 | 306,0 | HEMO | 0,20 | 26,24 | 35 | 94 |
| 11 C | 1 A | 92,3 | 5 B | 0,10 | 228,0 | DMAE* | 0,60 | 53,46 | 70 | 96 |
| 12 C | 1 A | 92,3 | 6 B | 0,20 | 429,8 | DEAB | 0,20 | 29,02 | 40 | 97 |
| 13 C | 2 A | 99,5 | 7 B | 0,25 | 88,0 | DEAE | 0,10 | 11,72 | 35 | 96 |
| 14 C | 1 A | 92,3 | 8 B | 0,20 | 230,4 | DEADG | 0,16 | 25,76 | 36 | 98 |
| 15 C | 3 A | 88,7 | 9 B | 0,17 | 346,8 | DMAE* | 0,25 | 22,27 | 42 | 95 |
| 16 C | 1 A | 92,3 | 10 B | 0,14 | 320,6 | DMAP | 0,32 | 32,99 | 46 | 98 |
| 17 C | 1 A | 92,3 | 11 B | 0,12 | 285,2 | DEAE | 0,40 | 46,88 | 52 | 97 |

Tabelle 3 - Fortsetzung

| Beispiel | Polyacrylat | | Fettalkoholalkoxylate | | | Dialkylaminoalkanol | | | Substitutionsgrad | |
|---|---|---|---|---|---|---|---|---|---|---|
| Nr. | Beispiel Nr. | Menge [ g ] | Beispiel Nr. | Menge [Mol] | Menge [ g ] | Art | Menge [Mol] | Menge [ g ] | theor. [ % ] | prakt. [ % ] |
| 18 C | 1 A | 92,3 | 12 B | 0,05 | 195,2 | DMAP | 0,05 | 5,16 | 10 | 93 |
| 19 C | 4 A | 86,7 | 13 B | 0,23 | 512,0 | DMAP | 0,20 | 20,62 | 43 | 96 |
| 20 C | 1 A | 92,3 | 14 B | 0,25 | 241,8 | DEADG | 0,13 | 20,93 | 38 | 98 |
| 21 C | 5 A | 148,2 | 15 B | 0,25 | 237,3 | DEAB | 0,20 | 29,02 | 45 | 98 |
| 22 C | 4 A | 86,7 | 16 B | 0,10 | 197,0 | DEAE | 0,15 | 17,58 | 25 | 97 |
| 23 C | 6 A | 184,6 | 17 B | 0,18 | 118,8 | DEAE | 0,40 | 46,88 | 58 | 95 |
| 24 C | 1 A | 92,3 | 18 B | 0,14 | 357,0 | DMAE* | 0,26 | 23,16 | 40 | 97 |
| 25 C | 1 A | 92,3 | 19 B | 0,07 | 138,7 | DEADG | 0,08 | 12,88 | 15 | 98 |
| 26 C | 6 A | 184,6 | 9 B | 0,21 | 428,4 | DEAB | 0,25 | 36,28 | 46 | 95 |
| 27 C | 1 A | 92,3 | 10 B | 0,18 | 412,2 | DMAP | 0,18 | 18,56 | 36 | 96 |
| 28 C | 1 A | 92,3 | 11 B | 0,20 | 475,4 | DEADG | 0,30 | 48,30 | 50 | 97 |
| 29 C | 1 A | 92,3 | 12 B | 0,16 | 390,4 | DMAE* | 0,20 | 17,82 | 36 | 96 |
| 30 C | 5 A | 148,2 | 13 B | 0,20 | 445,2 | DMAE* | 0,30 | 26,73 | 50 | 98 |
| 31 C | 2 A | 99,5 | 14 B | 0,27 | 261,1 | DEAE | 0,45 | 52,74 | 70 | 94 |
| 32 C | 1 A | 92,3 | 20 B | 0,20 | 960,0 | DEAE | 0,15 | 17,58 | 35 | 92 |
| 33 C | 1 A | 92,3 | 1 B/13 B | 0,1/0,15 | 583,9 | DEAE | 0,25 | 29,30 | 50 | 96 |
| 34 C | 6 A | 184,6 | 9 B/18 B | 0,1/0,1 | 459,0 | DEAE | 0,20 | 23,44 | 40 | 97 |

* Katalysator 10 Mol-% $NaOCH_3$

Legende:

| | | |
|---|---|---|
| DMAE | = | Dimethylaminoethanol |
| DEAE | = | Diethylaminoethanol |
| DMAP | = | Dimethylaminopropanol |
| DEADG | = | Diethylaminodiglycol |
| DEAB | = | Diethylaminobutanol |
| HEMO | = | Hydroxyethylmorpholin |

Beispiel 1 D

Quaternierung eines Umesterungsproduktes aus Polymethylacrylat, Stearyloxyethoxylat und Diethylaminoethanol

Bei dem gemäß Beispiel 1 C erhaltenen Produkt wird das als Lösungsmittel eingesetzte aromatenfreie Benzin durch Destillation entfernt und danach n-Propanol im Gewichtsverhältnis 1 : 1 zum Umesterungsprodukt zugegeben. Die erhaltene Lösung wird unter Stickstoff in einem Autoklaven auf 120 °C erhitzt. Durch Einleitung von Methylchlorid wird ein Druck von ca. 4 bar eingestellt, der während der Quaternierung durch Nachdosieren konstant gehalten wird.

Aus der Bestimmung des Chloridgehaltes und der Aminzahl ergibt sich ein Umsatz von 98 bzw. 99 %.

Beispiele 2 D bis 34 D

Quaternierung von Umesterungsprodukten aus verschiedenen Polyacrylaten, Fettalkoholalkoxylaten und tertiäre Stickstoffgruppen enthaltenden Alkanolen

Es wird grundsätzlich verfahren wie in Beispiel 1 D mit der Ausnahme, daß bei der Quaternierung die Umesterungsprodukte aus den Beispielen 1 C bis 34 C und in einigen Fällen Benzylchlorid verwendet werden. In Tabelle 4 werden die Umsätze bei der Quaternierung anhand der Chlorwerte und der Aminzahlen gezeigt.

Tabelle 4

| Beispiel Nr. | Umesterungsprodukt aus Beispiel Nr. | Quaternierungsagens Art | Quaternierungsagens Menge [ g ] | Umsatz aus $Cl^-$ [ % ] | Umsatz aus $N_{tert.}$ [ % ] |
|---|---|---|---|---|---|
| 1 D | 1 C | MC | 20,20 | 98 | 99 |
| 2 D | 2 C | MC | 12,63 | 99 | 98 |
| 3 D | 3 C | MC | 2,53 | 97 | 97 |
| 4 D | 4 C | MC | 7,58 | 99 | 99 |
| 5 D | 5 C | MC | 16,67 | 97 | 100 |
| 6 D | 6 C | MC | 22,73 | 99 | 98 |
| 7 D | 7 C | BC | 12,66 | 100 | 99 |
| 8 D | 8 C | MC | 25,25 | 98 | 97 |
| 9 D | 9 C | BC | 37,98 | 95 | 96 |
| 10 D | 10 C | MC | 10,10 | 100 | 99 |
| 11 D | 11 C | MC | 30,30 | 99 | 100 |
| 12 D | 12 C | MC | 10,10 | 98 | 97 |
| 13 D | 13 C | MC | 5,05 | 99 | 98 |
| 14 D | 14 C | BC | 20,26 | 98 | 98 |
| 15 D | 15 C | MC | 12,63 | 97 | 96 |
| 16 D | 16 C | BC | 40,51 | 99 | 100 |
| 17 D | 17 C | MC | 20,20 | 99 | 98 |
| 18 D | 18 C | BC | 6,33 | 98 | 98 |
| 19 D | 19 C | MC | 10,10 | 98 | 99 |
| 20 D | 20 C | MC | 6,57 | 100 | 98 |
| 21 D | 21 C | BC | 25,32 | 97 | 99 |
| 22 D | 22 C | MC | 7,58 | 98 | 97 |
| 23 D | 23 C | BC | 50,64 | 99 | 100 |
| 24 D | 24 C | BC | 32,92 | 99 | 98 |
| 25 D | 25 C | MC | 4,04 | 97 | 98 |

Tabelle 4 - Fortsetzung

| Beispiel Nr. | Umesterungsprodukt aus Beispiel Nr. | Quaternierungsagens Art | Quaternierungsagens Menge [ g ] | Umsatz aus $Cl^-$ [ % ] | Umsatz aus $N_{tert.}$ [ % ] |
|---|---|---|---|---|---|
| 26 D | 26 C | MC | 12,63 | 98 | 99 |
| 27 D | 27 C | BC | 22,79 | 97 | 97 |
| 28 D | 28 C | BC | 37,98 | 97 | 97 |
| 29 D | 29 C | BC | 25,32 | 98 | 96 |
| 30 D | 30 C | MC | 15,15 | 100 | 100 |
| 31 D | 31 C | MC | 22,73 | 97 | 99 |
| 32 D | 32 C | MC | 7,58 | 96 | 98 |
| 33 D | 33 C | BC | 31,65 | 95 | 97 |
| 34 D | 34 C | MC | 10,10 | 98 | 99 |

Legende:

MC = Methylchlorid
BC = Benzylchlorid

Beispiel 1 E

Protonierung eines Umesterungsproduktes aus Polymethylacrylat, Stearyloxyethoxolat und Diethylaminoethanol

Bei dem gemäß Beispiel 1 C erhaltenen Produkt wird das als Lösungsmittel eingesetzte aromatenfreie Benzin durch Destillation entfernt. Danach werden 7,13 g auf 100 g Umesterungsprodukt (= 90 Mol-%, bezogen auf tertiären Stickstoff) Propionsäure zugegeben und innerhalb von 1 h verrührt. Der anschließend ermittelte pH-Wert einer 10 %igen wäßrigen Lösung beträgt 5,2.

Beispiele 2 E bis 33 E

Es wird grundsätzlich verfahren wie in Beispiel 1 E mit der Ausnahme, daß bei der Protonierung Umesterungsprodukte aus den Beispielen 1 C bis 34 C mit unterschiedlichen Carbonsäuren bzw. anorganischen Säuren umgesetzt werden. In der Tabelle 5 werden das eingesetzte Umesterungsprodukt, die Art der Säure und die Menge in g sowie der resultierende pH-Wert angegeben.

Tabelle 5

| Beispiel Nr. | Umesterungsprodukt aus Beispiel Nr. | Protonierungsagens | | pH-Wert |
|---|---|---|---|---|
| | | Art | Menge [ g ]* | |
| 1 E | 1 C | PS | 26,64 | 5,20 |
| 2 E | 2 C | PS | 16,65 | 5,40 |
| 3 E | 3 C | PS | 3,33 | 6,50 |
| 4 E | 4 C | PS | 9,99 | 5,60 |
| 5 E | 5 C | PS | 21,98 | 5,30 |
| 6 E | 6 C | ES | 24,30 | 5,10 |
| 7 E | 7 C | ES | 6,00 | 5,80 |
| 8 E | 8 C | OES | 126,90 | 8,20 |
| 9 E | 9 C | PS | 19,98 | 5,40 |
| 10 E | 10 C | PS | 13,32 | 5,50 |
| 11 E | 11 C | OES | 152,28 | 8,10 |
| 12 E | 12 C | ES | 10,80 | 5,20 |
| 13 E | 13 C | PS | 6,66 | 6,20 |
| 14 E | 14 C | PS | 10,66 | 6,10 |
| 15 E | 15 C | PS | 16,65 | 5,70 |
| 16 E | 16 C | PS | 21,31 | 5,50 |
| 17 E | 17 C | PHS | 35,28 | 4,70 |
| 18 E | 18 C | PS | 3,33 | 6,80 |
| 19 E | 19 C | PHS | 17,64 | 4,90 |
| 20 E | 20 C | PS | 8,66 | 5,90 |

Tabelle 5

| Beispiel Nr. | Umesterungsprodukt aus Beispiel Nr. | Protonierungsagens Art | Menge [ g ]* | pH-Wert |
|---|---|---|---|---|
| 21 E | 21 C | PS | 13,32 | 5,50 |
| 22 E | 22 C | PS | 9,99 | 5,80 |
| 23 E | 23 C | OES/PHS 1:1 | 68,40 | 5,40 |
| 24 E | 24 C | PS | 17,32 | 5,50 |
| 25 E | 25 C | PS | 5,33 | 6,60 |
| 26 E | 26 C | PS | 16,65 | 5,50 |
| 27 E | 27 C | PS | 11,99 | 5,70 |
| 28 E | 28 C | PS | 19,98 | 5,40 |
| 29 E | 29 C | PS | 13,32 | 5,80 |
| 30 E | 30 C | PS | 19,98 | 5,40 |
| 31 E | 31 C | PS | 29,97 | 5,30 |
| 32 E | 33 C | PS | 16,65 | 5,50 |
| 33 E | 34 C | PS | 13,32 | 5,40 |

* 90 Mol-% des eingesetzten Aminoalkanols

Legende:

PS = Propionsäure
ES = Essigsäure
PHS = Phosphorsäure
OES = Ölsäure
OES/PHS 1:1 = Ölsäure/Phosphorsäure im Molverhältnis 1 : 1

Anwendungstechnische Beispiele

Herstellung und überprüfung von Haarshampoos unter Verwendung von Umesterungsprodukten aus verschiedenen Beispielen mit Quatgruppen und nichtionischen Gruppen

| Konditioniershampoo | |
|---|---|
| Umesterungsprodukt 2 D (Tabelle 4) | 2,0 % |
| Natriumlaurylethersulfat | 12,0 % |
| Cocoamidopropyl-Betain | 3,0 % |
| Wasser | 83,0 % |

Zur Herstellung des Haarbehandlungsmittels werden die Bestandteile in der aufgeführten Reihenfolge gemischt zusammengegeben. Jede Mischung muß vor der Zugabe weiterer Komponenten klar gelöst sein. Der pH-Wert dieser Mischungen beträgt etwa 5,5 bis 6,0.

In der praktischen Anwendung ergibt sich gegenüber Shampooformulierungen mit polyquaternären Verbindungen aus dem Stand der Technik neben einer guten antistatischen Wirkung und einer verbesserten Naßkämmbarkeit eine erhöhte Viskosität, wodurch auf ein zusätzliches Mittel zur Einstellung des Fließverhaltens verzichtet werden kann.

Bei Beibehaltung der vorgenannten Rezeptur werden bei Variation der Konzentration der Umesterungsprodukte 1 D und 2 D in der obigen Formulierung folgende Viskositäten erreicht:

Tabelle 6

| Viskositäten der Shampooformulierungen [ mPas ] | | |
|---|---|---|
| Konzentration [Gew.-%] | Polyacrylatderivat Beispiel Nr. | |
| | 1 D | 2 D |
| 0,0 | 40 | 40 |
| 0,5 | 500 | 2000 |
| 1,0 | 1380 | 2800 |
| 2,0 | 2800 | 4800 |
| 3,0 | 2800 | 4300 |
| 5,0 | 1850 | 3250 |
| 10,0 | 625 | 1080 |

Der Vergleich unterschiedlicher Modifizierungstypen macht deutlich, daß die verdickende Wirkung mit steigendem Anteil ethoxylierten Stearylalkohols und sinkendem Anteil Quatgruppen zunimmt. In der obengenannten Rezeptur werden verschieden modifizierte Acrylatderivate in einer Konzentration von 2 Gew.-% in jeweils einer Shampooformulierung eingesetzt und die Viskositäten der Shampooformulierungen miteinander verglichen. Bei dem Umesterungsprodukt Nr. 2 E handelt es sich um einen aminofunktionellen Typ, der nur bei saurem pH-Wert in protonierter Form vorliegt. In der folgenden Tabelle 7 sind die Viskositätsdaten zusammengestellt.

Tabelle 7

| Polyacrylatderivat Beispiel Nr. | Viskosität der Shampooformulierung [ mPas ] |
|---|---|
| 1 D | 2800 |
| 2 D | 4800 |
| 3 D | 15000 |
| 4 D | 9500 |
| 5 D | 6000 |
| 6 D | 6500 |
| 2 E | 45000 |

## Patentansprüche

1. Polyacrylsäureester mit Ammonium- und langkettigen Kohlenwasserstoffoxy-Gruppen, erhältlich durch Umesterung von durch radikalische Polymerisation erhaltenen Polyacrylsäurealkylestern, deren Alkylreste 1 bis 8 Kohlenstoffatome aufweisen, wobei bis zu 50 % der Acrylsäureester durch die entsprechenden Methacrylsäureester ersetzt sein können, mit
   a) tertiären Aminen der allgemeinen Formel

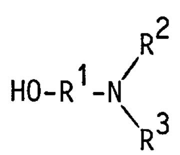

oder

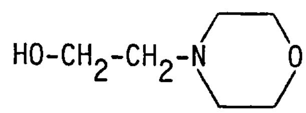

$R^1$ = zweiwertiger Rest der allgemeinen Formel $-(CH_2)_q-$,

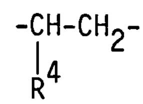

oder $-(C_nH_{2n}O)_m-C_pH_{2p}-$

$R^4$ = Alkylrest mit 1 bis 16 Kohlenstoffatomen,
q = 2, 3 oder 4,
n = 2, 3 oder 4,
m = 1 bis 20,
p = 2, 3 oder 4,
$R^2$, $R^3$ = jeweils Alkylrest mit 1 bis 18 Kohlenstoffatomen, und

b) Polyoxyalkylenmonoolen der allgemeinen durchschnittlichen Formel

$R^5O-(C_rH_{2r}O-)_sH$

$R^5$ = Alkylrest mit 8 bis 30 Kohlenstoffatomen, der im Polymeren gleich oder verschieden sein kann, oder Alkenylrest mit 8 bis 22 Kohlenstoffatomen, oder Mono- oder Dialkylphenylrest mit 6 bis 16 Kohlenstoffatomen je Alkylrest, wobei bis zu 40 % der Reste $R^5$ durch Alkylreste mit 1 bis 4 Kohlenstoffatomen ersetzt sein können,
r = 2, 3 oder 4 und im durchschnittlichen Molekül 2,0 bis 2,5,
s = 10 bis 100,

wobei das molare Verhältnis der tertiären Amine zu den Polyoxyalkylenmonoolen 1 : 9 bis 9 : 1 beträgt, in solchen Mengen, daß 5 bis 70 % der Estergruppen umgeestert werden, in Gegenwart eines an sich bekannten Umesterungskatalysators, bei Temperaturen von 70 bis 170 °C, gegebenenfalls in Gegenwart eines Lösungsmittels, und anschließende (1) Quaternierung der Umesterungsprodukte mit Alkyl- oder Arylhalogeniden der allgemeinen Formel $R^6X$, wobei $R^6$ ein Alkylrest mit 1 bis 4 Kohlenstoffatomen oder ein Benzylrest und X ein Halogenrest ist, oder mit Dimethyl- oder Diethylsulfat in an sich bekannter Weise bei Temperaturen von 20 bis 140 °C und gegebenenfalls erhöhtem Druck oder (2) Protonierung der Umesterungsprodukte durch Umsetzung mit anorganischen oder organischen Säuren.

**2.** Polyacrylsäureester nach Anspruch 1, dadurch gekennzeichnet, daß die Umesterung mit solchen Mengen der Verbindungen a) und b) durchgeführt wird, daß 20 bis 50 % der Estergruppen umgeestert werden.

**3.** Polyacrylsäureester nach Anspruch 1 oder 2, dadurch gekennzeichnet, daß das molare Verhältnis der Verbindungen a) zu b) 1 : 3 bis 3 : 1 beträgt.

**4.** Polyacrylsäureester nach einem oder mehreren der vorhergehenden Ansprüche, dadurch gekennzeichnet, daß $R^5$ ein Alkylrest mit 14 bis 22 Kohlenstoffatomen ist.

**5.** Polyacrylsäureester nach einem oder mehreren der vorhergehenden Ansprüche, dadurch gekennzeichnet, daß $R^5$ ein Alkenylrest mit 8 bis 18 Kohlenstoffatomen ist.

**6.** Polyacrylsäureester nach einem oder mehreren der vorhergehenden Ansprüche, dadurch gekennzeichnet, daß $R^5$ ein Monoalkylphenylrest ist, dessen Alkylgruppe 6 bis 12 Kohlenstoffatome aufweist.

**7.** Polyacrylsäureester nach einem oder mehreren der vorhergehenden Ansprüche, dadurch gekennzeichnet, daß r einen mittleren Wert von 2,0 bis 2,3 hat.

**8.** Verwendung der Polyacrylsäureester nach einem oder mehreren der vorhergehenden Ansprüche als wäßrige Lösungen verdickende Antistatika für kosmetische, insbesondere haarpflegende Zubereitungen.

## Claims

**1.** Polyacrylic acid esters with ammonium and long-chain hydrocarbonoxy groups which are obtainable by transesterification of polyacrylic acid alkyl esters obtained by free radical polymerization, the alkyl radicals of which have 1 to 8 carbon atoms, it being possible for up to 50 % of the acrylic acid esters to be replaced by the corresponding methacrylic acid esters, with

a) tertiary amines of the general formula

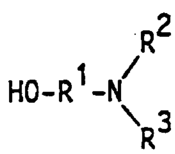

or

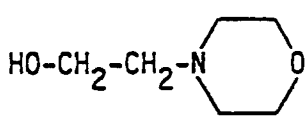

$R^1$ = a divalent radical of the general formula $-(CH_2)_q-$,

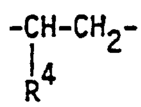

or $-(C_nH_{2n}O)_m-C_pH_{2p}-$

$R^4$ = an alkyl radical having 1 to 16 carbon atoms,

q = 2, 3 or 4,

n = 2, 3 or 4,

m = 1 to 20,

p = 2, 3 or 4,

$R^2$,$R^3$ = in each case an alkyl radical having 1 to 18 carbon atoms, and

b) polyoxyalkylene-monools of the general average formula

$R^5O\text{-}(C_rH_{2r}O\text{-})_sH$

$R^5$ = an alkyl radical having 8 to 30 carbon atoms, which can be identical or different in the polymer, or an alkenyl radical having 8 to 22 carbon atoms or a mono- or dialkylphenyl radical having 6 to 16 carbon atoms per alkyl radical, it being possible for up to 40 % of the radicals $R^5$ to be replaced by alkyl radicals having 1 to 4 carbon atoms,

r = 2, 3 or 4, and in the average molecule 2.0 to 2.5,

s = 10 to 100,

the molar ratio of the tertiary amines to the polyoxyalkylene-monools being 1 : 9 to 9 : 1, in amounts such that 5 to 70 % of the ester groups are transesterified, in the presence of a transesterification catalyst which is known per se at temperatures of 70 to 170 °C, if appropriate in the presence of a solvent, and subsequent (1) quaternization of the transesterification products with alkyl or aryl halides of the general formula $R^6X$, wherein $R^6$ is an alkyl radical having 1 to 4 carbon atoms or a benzyl radical and X is a halogen radical, or with dimethyl or diethyl sulphate in a manner which is known per se at temperatures of 20 to 140 °C, and if appropriate under increased pressure, or (2) protonation of the transesterification products by reaction with inorganic or organic acids.

**2.** Polyacrylic acid eaters according to Claim 1, characterized in that the transesterification is carried out with amounts of compounds a) and b) such that 20 to 50 % of the ester groups are transesterified.

**3.** Polyacrylic acid esters according to Claim 1 or 2, characterized in that the molar ratio of the compounds a) to b) is 1 : 3 to 3 : 1.

**4.** Polyacrylic acid esters according to one or more of the preceding claims, characterized in that $R^5$ is an alkyl radical having 14 to 22 carbon atoms.

**5.** Polyacrylic acid esters according to one or more of the preceding claims, characterized in that $R^5$ is an alkenyl radical having 8 to 18 carbon atoms.

**6.** Polyacrylic acid esters according to one or more of the preceding claims, characterized in that $R^5$ is a monoalkylphenyl radical, the alkyl group of which has 6 to 12 carbon atoms.

**7.** Polyacrylic acid esters according to one or more of the preceding claims, characterized in that r has an average value of 2.0 to 2.3.

**8.** Use of the polyacrylic acid esters according to one or more of the preceding claims as antistatics which thicken aqueous solutions and are intended for cosmetic formulations, in particular hair care formulations.

## Revendications

**1.** Polyacrylates contenant des groupes d'ammonium et des groupes d'hydrocarbures et d'oxyalkylène à chaîne longue, qu'on peut obtenir par transestérification de polyacrylates d'alkyle obtenus par polymérisation radicalaire, dont les groupes alkyles comportent de 1 à 8 atomes de carbone, jusqu'à 50 % des acrylates pouvant être remplacés par les méthacrylates correspondants, avec

a) des amines tertiaires répondant à la formule générale :

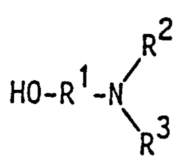

ou

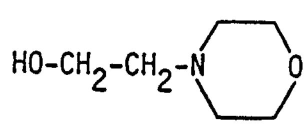

$R^1$ = reste bivalent répondant à la formule générale $-(CH_2)_q-$,

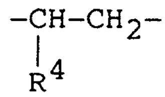

ou $-(C_nH_{2n}O)_m-C_pH_{2p}-$

$R^4$ = reste alkyle ayant de 1 à 16 atomes de carbone,
q = 2, 3 ou 4,
n = 2, 3 ou 4,
m = 1 à 20,
p = 2, 3 ou 4,
$R^2$, $R^3$ = chacun un reste alkyle ayant de 1 à 18 atomes de carbone, et

b) des polyoxyalkylène-monools répondant à la formule générale moyenne

$R^5O-(C_rH_{2r}O-)_sH$

$R^5$ = reste alkyle ayant de 8 à 30 atomes de carbone, qui peuvent être identiques ou différents dans le polymère, ou reste alkényle ayant de 8 à 22 atomes de carbone, ou reste mono- ou dialkylphényle ayant de 6 à 16 atomes de carbone par reste alkyle, jusqu'à 40 % des restes $R^5$ pouvant être remplacés par des restes alkyles ayant de 1 à 4 atomes de carbone,
r = 2, 3 ou 4, et vaut de 2,0 à 2,5 dans la molécule moyenne,
s = 10 à 100,

le rapport molaire des amines tertiaires aux polyoxyalkylène-monools valant de 1:9 à 9:1,
en quantités telles que 5 à 70 % des groupes esters sont transestérifiés, en présence d'un catalyseur de transestérification connu en soi, à des températures de 70 à 170 °C, éventuellement en présence d'un solvant, suivie de (1) une quaternisation des produits de transestérification avec des halogénures d'alkyle ou d'aryle répondant à la formule générale $R^6X$, où $R^6$ est un reste alkyle ayant de 1 à 4 atomes de carbone ou un reste benzyle, et X est un reste d'halogène, ou avec un diméthyl- ou diéthylsulfate, d'une manière connue en soi, à des températures de 20 à 140 °C et éventuellement à une pression augmentée, ou (2) une protonation des produits de transestérification, par réaction avec des acides minéraux ou des acides organiques.

**2.** Polyacrylates selon la revendication 1, caractérisés en ce qu'on effectue la transestérification avec des quantités de composés a) et b) telles que 20 à 50 % des groupes esters sont transestérifiés.

**3.** Polyacrylates selon la revendication 1 ou 2, caractérisés en ce que le rapport molaire des composés a) à b) est de 1:3 à 3:1.

**4.** Polyacrylates selon une ou plusieurs des revendications précédentes, caractérisés en ce que $R^5$ est un reste alkyle ayant de 14 à 22 atomes de carbone.

**5.** Polyacrylates selon une ou plusieurs des revendications précédentes, caractérisés en ce que $R^5$ est un reste alkényle ayant de 8 à 18 atomes de carbone.

**6.** Polyacrylates selon une ou plusieurs des revendications précédentes, caractérisés en ce que $R^5$ est un reste monoalkylphényle dont le groupe alkyle contient de 6 à 12 atomes de carbone.

**7.** Polyacrylates selon une ou plusieurs des revendications précédentes, caractérisés en ce que r a une valeur moyenne de 2,0 à 2,3.

**8.** Utilisation des polyacrylates selon une ou plusieurs des revendications précédentes sous la forme d'agents antistatiques épaississant des solutions aqueuses pour des préparations cosmétiques, en particulier pour les soins capillaires.